# EUROPÄISCHE PATENTSCHRIFT

(11) **EP 3 116 589 B1**
(45) Veröffentlichungstag und Bekanntmachung des Hinweises auf die Patenterteilung: **15.07.2020**
(21) Anmeldenummer: 15709931.8
(22) Anmeldetag: 13.03.2015
(51) Int. Cl.: A61M 39/24

(54) **RÜCKSCHLAGVENTILANORDNUNG, MEDIZINISCHE FUNKTIONSVORRICHTUNG UND EINE BLUTBEHANDLUNGSVORRICHTUNG**
CHECK VALVE ARRANGEMENT, MEDICAL FUNCTION DEVICE, AND BLOOD TREATMENT DEVICE
SOUPAPE DE RETENUE, DISPOSITIF FONCTIONEL MÉDICAL ÉT DISPOSITIV DU TRAITEMENT SU SANG

(30) Priorität: 14.03.2014 DE 102014103489
(43) Veröffentlichungstag der Anmeldung: 18.01.2017
(73) Patentinhaber: Fresenius Medical Care Deutschland GmbH, 61352 Bad Homburg (DE)
(72) Erfinder: GÖSSMANN, Stephan, 61381 Friedrichsdorf (DE); HÄCKER, Jürgen, 61267 Neu-Anspach (DE); MÜLLER, Ralf, 61350 Bad Homburg (DE)
(74) Vertreter: Bobbert & Partner Patentanwälte PartmbB
(86) Internationale Anmeldenummer: PCT/EP2015/055278
(87) Internationale Veröffentlichungsnummer: WO 2015/136067

(56) Entgegenhaltungen:
- WO-A1-2010/102784
- DE-A1-102009 024 469
- US-A- 5 954 313
- US-A1- 2010 274 169

## Beschreibung

Die vorliegende Erfindung betrifft eine Rückschlagventilanordnung gemäß Anspruch 1. Sie betrifft zudem eine medizinische Funktionsvorrichtung gemäß Anspruch 12 und eine Blutbehandlungsvorrichtung gemäß Anspruch 16.

US 2010/274169 A1 offenbart eine Rückschlagventilanordnung mit Zulauf und Ablauf für medizinische Fluide, die in einem Ausgangszustand ein Durchströmen des Arbeitsfluids in beiden Durchströmungsrichtungen zulässt, jedoch in einem Betriebszustand nur eine Durchströmungsrichtung zulässt. Die Zustandsüberführung erfolgt durch Kraftaufbringung bzw. Wegeinprägung auf den Ventilkörper.

Eine Aufgabe der vorliegenden Erfindung ist es, eine weitere Rückschlagventilanordnung vorzuschlagen. Zudem sollen geeignete Funktionsvorrichtungen und Behandlungsvorrichtungen angegeben werden.

Die erfindungsgemäße Aufgabe wird durch eine Rückschlagventilanordnung mit den Merkmalen des Anspruchs 1 gelöst. Sie wird ferner gelöst mittels der medizinischen Funktionsvorrichtung mit den Merkmalen des nebengeordneten Anspruchs 12 sowie der Blutbehandlungsvorrichtung mit den Merkmalen des nebengeordneten Anspruchs 16.

Erfindungsgemäß wird somit eine Rückschlagventilanordnung mit einem Zulauf und einem Ablauf für medizinische Fluide, insbesondere ein Arbeitsfluid, vorgeschlagen, welche einen Ventilkörper aufweist. Die Rückschlagventilanordnung lässt in einem Ausgangszustand ein Durchströmen eines Arbeitsfluids und/oder eines Sterilisationsfluids durch die Rückschlagventilanordnung in beiden Durchströmungsrichtungen, also vom Zulauf zum Ablauf und umgekehrt, zu. Ferner lässt die Rückschlagventilanordnung in einem Betriebszustand nur eine Durchströmungsrichtung, also vom Zulauf zum Ablauf des Arbeitsfluids zu.

Die Rückschlagventilanordnung weist eine Stift-Stiftaufnahme-Verbindung (oder Stift-Aufnahme-Verbindung) auf. Diese ist derart ausgestaltet, dass die Rückschlagventilanordnung durch Kraftaufbringung auf die Stift-Stiftaufnahme-Verbindung oder auf den Stift und/oder durch eine Wegeinprägung auf die Stift-Stiftaufnahme-Verbindung oder auf den Stift - vorzugsweise jeweils in Längsrichtung von Stift und Stiftaufnahme - vom Ausgangszustand in den Betriebszustand überführbar ist.

Das Überführen kann nach Aufbringen oder mittels Aufbringens einer Kraft auf die Stift-Stiftaufnahme-Verbindung, z. B. über die Folie im Ausgangszustand und einer hierdurch bewirkt werden.

Die erfindungsgemäße medizinische Funktionsvorrichtung weist wenigstens eine erfindungsgemäße Rückschlagventilanordnung auf.

Die erfindungsgemäße Blutbehandlungsvorrichtung, vorzugsweise ausgestaltet als eine Dialysiervorrichtung, weist einen Aufnahmeabschnitt zur Aufnahme einer erfindungsgemäßen medizinischen Funktionsvorrichtung auf.

Die die Blutbehandlungsvorrichtung weist eine bewegbare Begrenzungsvorrichtung, z. B. eine Tür, zum Begrenzen des Aufnahmeabschnitts auf. Die Begrenzungsvorrichtung ist ausgestaltet, um durch ihre Bewegung eine oder alle Rückschlagventilanordnungen der in den Aufnahmeabschnitt aufgenommenen medizinischen Funktionsvorrichtung aus dem Ausgangszustand in den Betriebszustand zu überführen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine beispielhafte erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

Erfindungsgemäße beispielhafte Ausführungsformen können eines oder mehrere der im Folgenden genannten Merkmale in beliebiger Kombination aufweisen.

Die Stiftaufnahme-Stift-Verbindung kann in erfindungsgemäßen beispielhaften Ausführungsformen die Kombination aus einer Stiftaufnahme (oder eines Stiftaufnahmeabschnitts) zum Aufnehmen eines Stifts und einem Stift sein oder eine solche aufweisen, wobei der Stift angeordnet ist, um sich in der Stiftaufnahme und relativ zu dieser bewegen zu können.

Die Rückschlagventilanordnung ist in einigen erfindungsgemäßen, beispielhaften Ausführungsformen ausgestaltet, um zusätzlich zum Ausgangszustand genau einen oder wenigstens einen Betriebszustand annehmen zu können.

In manchen erfindungsgemäßen, beispielhaften Ausführungsformen ist der Ausgangszustand die erste Funktionsstellung, welche dem Sterilisieren dient, und der Betriebszustand die zweite Funktionsstellung, welche eine Rückschlagfunktion zeigt.

In manchen erfindungsgemäßen, beispielhaften Ausführungsformen kann die Rückschlagventilanordnung alternativ oder ergänzend zur Stift-Stiftaufnahme-Verbindung eine Stift-Stiftaufnahme-Führung aufweisen.

In einigen erfindungsgemäßen, beispielhaften Ausführungsformen der Rückschlagventilanordnung ist die Stift-Stiftaufnahme-Verbindung derart ausgestaltet, dass die Rückschlagventilanordnung nach Kraftaufbringung auf die Stift-Stiftaufnahme-Verbindung und/oder nach einer Wegeinprägung auf die Stift-Stiftaufnahme-Verbindung auch nach Wegfall der Kraft in der Betriebsstellung verbleibt. Dieses Verbleiben zeichnet den hierin als Verrastung bezeichneten Zustand aus. Eine alternative Bezeichnung für diesen Zustand ist "remanent aktiviert".

In manchen erfindungsgemäßen, beispielhaften Ausführungsformen weist die Rückschlagventilanordnung im Ausgangszustand einen Bypass auf, mittels welchem das Sterilisationsfluid die Rückschlagventilanordnung durchströmen kann.

In einigen erfindungsgemäßen, beispielhaften Ausführungsformen weist die Rückschlagventilanordnung nur im Ausgangszustand einen Bypass zum Durchströmen des Sterilisationsfluids auf, nicht aber auch im Betriebszustand.

In bestimmten erfindungsgemäßen, beispielhaften Ausführungsformen weist die Rückschlagventilanordnung im Ausgangszustand einen Bypass, der nicht der Hauptdurchströmung des Arbeitsfluids dient, zum Durchströmen des Sterilisationsfluids auf.

In manchen erfindungsgemäßen, beispielhaften Ausführungsformen der Rückschlagventilanordnung ist die Stiftaufnahme der Stift-Stiftaufnahme-Verbindung der Bypass oder umfasst oder bildet zumindest bereichsweise den Bypass.

In gewissen erfindungsgemäßen, beispielhaften Ausführungsformen der Rückschlagventilanordnung ist die Stiftaufnahme ein Durchgangsloch oder ein Sacklock.

In bestimmten erfindungsgemäßen, beispielhaften Ausführungsformen der Rückschlagventilanordnung ist die Stiftaufnahme durch Bohren entstanden, in anderen durch Umspritzen.

In manchen erfindungsgemäßen, beispielhaften Ausführungsformen der Rückschlagventilanordnung weist die Stiftaufnahme ausschließlich oder in wenigstens einem Abschnitt hiervon einen runden Querschnitt auf.

In einigen erfindungsgemäßen, beispielhaften Ausführungsformen der Rückschlagventilanordnung ist der Stift der Stift-Stiftaufnahme-Verbindung innen hohl und umfasst oder bildet zumindest bereichsweise den Bypass.

In bestimmten erfindungsgemäßen, beispielhaften Ausführungsformen der Rückschlagventilanordnung ist der Stift der Stift-Stiftaufnahme-Verbindung durchgängig oder abschnittsweise hohl.

In manchen erfindungsgemäßen, beispielhaften Ausführungsformen der Rückschlagventilanordnung ist der Stift der Stift-Stiftaufnahme-Verbindung an einer oder beiden seiner Stirnflächen zumindest teilweise offen.

In einigen erfindungsgemäßen, beispielhaften Ausführungsformen der Rückschlagventilanordnung weist der Stift der Stift-Stiftaufnahme-Verbindung seitliche, also sich in seiner Mantelfläche befindliche Öffnungen auf.

In manchen erfindungsgemäßen, beispielhaften Ausführungsformen der Rückschlagventilanordnung ist die Stift-Stiftaufnahme-Verbindung eine Stift-Stiftaufnahme-Paarung.

In bestimmten erfindungsgemäßen, beispielhaften Ausführungsformen der Rückschlagventilanordnung dichtet der Stift der Stift-Stiftaufnahme-Verbindung zumindest bereichsweise auf seinem äußeren Umfang gegen den Ventilkörper ab.

In manchen erfindungsgemäßen, beispielhaften Ausführungsformen der Rückschlagventilanordnung hat der Stift im Bereich seines äußeren Umfangs "Spiel" für zumindest einen Teil der Bypassströmung.

In einigen erfindungsgemäßen, beispielhaften Ausführungsformen der Rückschlagventilanordnung weist der Ventilkörper sowohl den Stift als auch die Stiftaufnahme auf.

In bestimmten erfindungsgemäßen, beispielhaften Ausführungsformen weist die Rückschlagventilanordnung im Betriebszustand eine reibschlüssige Verbindung des Stifts mit wenigstens einem Abschnitt der Stiftaufnahme auf.

In gewissen erfindungsgemäßen, beispielhaften Ausführungsformen ist ein Außendurchmesser des Stifts - im Bereich seiner Verbindung mit (oder seiner Aufnahme in) der Stiftaufnahme - um ein Maß, welches hier als Übermaß bezeichnet werden kann, größer als ein Innendurchmesser der Stiftaufnahme, welches erforderlich ist, um - falls gewünscht - einen Reibschluss zwischen Stift und Stiftaufnahme zu erzielen.

In manchen beispielhaften Ausführungsformen der erfindungsgemäßen Rückschlagventilanordnung weist der Ventilkörper einen ersten Bereich mit Stiftaufnahme für den Stift bzw. die Stift-Stiftaufnahme-Verbindung und einen zweiten Bereich zum Abdichten eines Strömungswegs des Arbeitsfluids im Betriebszustand auf, wobei der zweite Bereich flexibler oder weicher als der erste Bereich ausgestaltet ist, oder der erste Bereich starrer als der zweite Bereich ist.

In einigen beispielhaften Ausführungsformen der erfindungsgemäßen Rückschlagventilanordnung ist der Ventilkörper oder ein Ventileinsatz aus Kunststoff oder weist Kunststoff auf.

In bestimmten erfindungsgemäßen, beispielhaften Ausführungsformen der Rückschlagventilanordnung ist der Ventilkörper oder der Ventileinsatz als 2-Komponenten-Spritzgußteil hergestellt.

In manchen erfindungsgemäßen, beispielhaften Ausführungsformen der Rückschlagventilanordnung ist die Stift-Stiftaufnahme-Verbindung ausgestaltet, um die Rückschlagventilanordnung mittels Reibschluss und/oder Formschluss im Betriebszustand zu halten.

In einigen erfindungsgemäßen, beispielhaften Ausführungsformen der Rückschlagventilanordnung ist die Stift-Stiftaufnahme-Verbindung ausgestaltet, um die Rückschlagventilanordnung allein mittels Reibschluss und/oder Formschluss im Betriebszustand zu halten.

In manchen erfindungsgemäßen, beispielhaften Ausführungsformen ist die medizinische Funktionsvorrichtung eine Blutkassette oder ein Blutschlauchsatz, insbesondere jeweils für die Dialyse, mit wenigstens einer Rückschlagventilanordnung.

In einigen beispielhaften Ausführungsformen der erfindungsgemäßen medizinischen Funktionsvorrichtung weist die Rückschlagventilanordnung einen Ventilsitz und optional eine Folie zum Abdichten des Ventilsitzes, des Arbeitsfluids und/oder des Sterilisationsfluids gegen die Umgebung auf.

In manchen erfindungsgemäßen, beispielhaften Ausführungsformen der medizinischen Funktionsvorrichtung ist der Stift mit dem Ventilsitz verbunden oder Teil hiervon. Die Stiftaufnahme ist relativ zu dem Stift verschiebbar und Teil des Ventilkörpers.

In einigen erfindungsgemäßen, beispielhaften Ausführungsformen der medizinischen Funktionsvorrichtung ist die Stiftaufnahme relativ zu dem Stift verschiebbar und Teil des Ventilkörpers unter Erzielen eines Reibschlusses zwischen Stift und Stiftaufnahme.

In bestimmten erfindungsgemäßen, beispielhaften Ausführungsformen ist die medizinische Funktionsvorrichtung mit wenigstens zwei Rückschlagventilanordnungen ausgestattet, welche angeordnet sind, um vorzugsweise gleichzeitig aus dem Ausgangszustand in den Betriebszustand überführbar oder als Rückschlagventil aktivierbar zu sein.

Einige oder alle erfindungsgemäßen Ausführungsformen können einen oder mehrere der oben oder im Folgenden genannten Vorteile aufweisen.

In bestimmten erfindungsgemäßen Ausführungsformen ist eine Dichtlippe aus einem Elastomer gefertigt. Aufgrund der Elastizität des Elastomers dient die Dichtlippe daher vorteilhaft auch einem Toleranzausgleich, ohne jedoch den Öffnungsdruck der Rückschlagventilanordnung durch eine höhere Vorspannung auf die Dichtlippe wesentlich zu verändern.

In einigen erfindungsgemäßen Ausführungsformen dienen Bereiche der Rückschlagventilanordnung, die aus Elastomer hergestellt sind, vorteilhaft dem Toleranzausgleich bei einem Montagevorgang der Rückschlagventilanordnung, insbesondere bei einem Verpressvorgang des Ventilkörpers und der Dichtlippe in einen Kassettenkörper. Beispielsweise kann bei dem Verpressvorgang eine Kraft auf einen Elastomerbereich ausgeübt werden, der mit der Dichtlippe verbunden ist. Dieser Elastomerbereich kann wiederum mit einem Ventilkörper aus einem Hartkunststoff verbunden sein, insbesondere mittels eines 2K-Kunststoffteils (2-Komponenten-Spritzgußteil). Bei dem Verpressvorgang kann schließlich vorteilhaft eine Kraft (vorzugsweise ausschließlich) auf das Elastomer ausgeübt werden, ohne den Ventilkörper zu beschädigen. Dies ist ein vorteilhafter weiterer Toleranzausgleich mittels des Elastomers.

Bei manchen erfindungsgemäßen Rückschlagventilanordnungen kann ihre Aktivierung, d. h. ihre Rückschlagfunktion vorteilhaft, auch nach dem Abrüsten der medizinischen Funktionsvorrichtung oder des beispielsweise als medizinische Funktionsvorrichtung ausgestalteten Disposables, erhalten bleiben. Damit kann das Austreten von Flüssigkeit auch beim Abrüsten mittels der erfindungsgemäßen, remanent (oder dauerhaft) aktivierten Rückschlagventilanordnung verhindert werden. Hierdurch können am Disposable zwei Schlauchklemmen und eine zu ihrer Betätigung erforderliche Tätigkeit des Anwenders eingespart werden.

Weiterhin bedarf es keiner Verschlusshülse im Substituatkonnektor (wie in der WO 2010/121819 A1 der Anmelderin der vorliegenden Anmeldung für beispielhafte Funktionsvorrichtungen (dort: eine Blutkassette) beschrieben) und eines dazugehörigen Arbeitshubs der Maschine, um einen Schutz vor Kontamination mittels aus der medizinischen Funktionsvorrichtung, beispielsweise einer Blutkassette, austretender Flüssigkeiten zu vermeiden.

Die erfindungsgemäßen Rückschlagventilanordnungen können vorteilhaft die erforderliche Robustheit gegenüber vorzeitiger Aktivierung in der Produktion vor der Sterilisation aufbieten.

Des Weiteren können die erfindungsgemäßen Rückschlagventilanordnungen Vorteile bei der automatischen Herstellung, der sicheren Sterilisation und der Qualitätssteigerung der Rückschlagventilfunktionen bieten. Hierzu zählen ein sicheres Verbleiben in der Ausgangsstellung, bis zum Abschluss der Gassterilisation, geringe und definierte Eigenschaftsänderungen durch die Gassterilisation insbesondere des Öffnungsdrucks, die Minimierung von Eigenschaftsänderungen der eingebauten Rückschlagventilanordnungen durch mechanische, thermische oder bestrahlungsinduzierte Belastung während der Lagerung und des Transports, welche den Vorgang der sicheren und präzisen Aktivierung im Rahmen des Aufrüstvorgangs beeinträchtigen könnten, Vermeidung des bisher tolerierten Überstandes der folienseitigen Stirnflächen der Rückschlagventilanordnungen über die Folienebene der Kassette (Rückschlagventilanordnungen verursachen lokal Beulen in der Folie) zur Gewährleistung einer sicher herstellbaren Folienschweißnaht, ausgehend von einer durchgehend eben auf der Kassette aufliegenden Folie, toleranzen-tolerantere Gestaltung der Einzelteile und ihres Zusammenwirkens sowohl bei der Montage wie im Zusammenwirken mit der Behandlungsmaschine bei gleichzeitig hoher Reproduzierbarkeit der Dichtheit, des Öffnungsdrucks und der Durchfluss-Druckabfall-Kennlinie und dadurch sichere Funktion auch bei Großserienproduktion.
- **Fig. 1a**: zeigt eine Schnittdarstellung der erfindungsgemäßen Rückschlagventilanordnung einer ersten Ausführungsform im Ausgangszustand;
- **Fig. 1b**: zeigt eine Schnittdarstellung der erfindungsgemäßen Rückschlagventilanordnung der Fig. 1a im Betriebszustand;
- **Fig. 2a**: zeigt eine Schnittdarstellung der erfindungsgemäßen Rückschlagventilanordnung einer zweiten Ausführungsform in Explosionsdarstellung;
- **Fig. 2b**: zeigt eine Schnittdarstellung der Rückschlagventilanordnung der Fig. 2a im Ausgangszustand;
- **Fig. 2c**: zeigt eine Schnittdarstellung der Rückschlagventilanordnung der Fig. 2a und 2b im Betriebszustand; und
- **Fig. 3**: zeigt schematisch stark vereinfacht eine Schnittdarstellung der erfindungsgemäßen Blutbehandlungsvorrichtung 300.

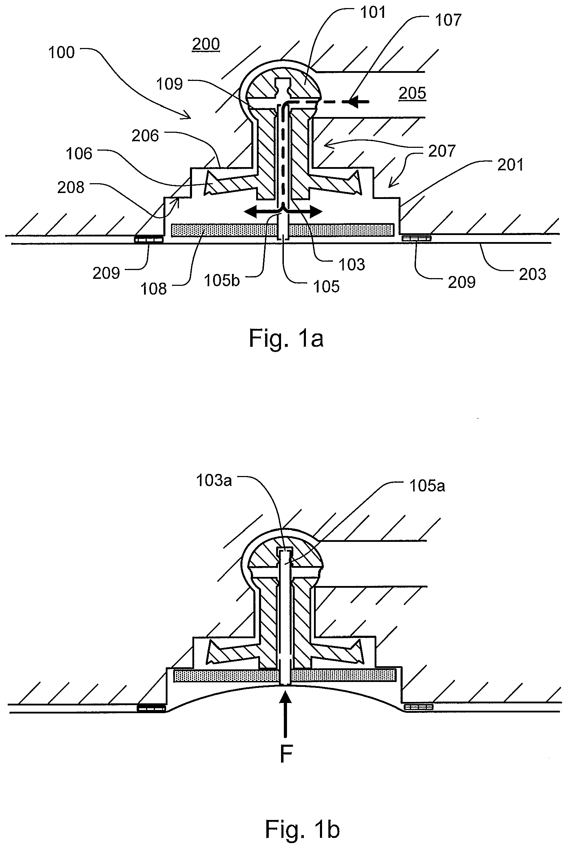

**Fig. 1a** zeigt eine erfindungsgemäße Rückschlagventilanordnung 100 in einer ersten Ausführungsform als Teil einer erfindungsgemäßen medizinischen Funktionsvorrichtung 200, hier beispielhaft in Gestalt einer Blutkassette.

Die Blutkassette 200 weist einen Kassettenkörper 201, ausgestaltet als ein Hartteil, und eine Folie 203 auf. Sie weist ferner einen Zulauf 205 für ein Arbeitsfluid (beispielsweise Blut) und einen - da rechtwinklig zum Zulauf 205 aus der Zeichenebene austretend - nicht dargestellten Ablauf. Im Bereich ihres Kassettenkörpers 201 weist die Blutkassette 200 einen Ventilsitz 207 auf.

Befestigungen 209 halten die Folie 203 am Kassettenkörper 201. Die Befestigung kann eine Klebeverbindung, eine Pressverbindung, eine Schweißverbindung oder dergleichen sein.

Im Ventilsitz 207 findet sich die Rückschlagventilanordnung 100. Diese weist einen Ventilkörper 101 mit einer Stiftaufnahme 103 und einen in die Stiftaufnahme 103 eingeschobenen Stift 105 auf. Stiftaufnahme 103 und Stift 105 ergeben zusammen eine Stiftaufnahme-Stift-Verbindung, in welcher sich der Stift 105 relativ zur Stiftaufnahme 103 bewegen kann und dabei von dieser geführt wird.

Der Ventilkörper 101 weist ferner eine umlaufende Dichtlippe 106 auf, welche gegen eine Anschlagfläche 206 des Ventilsitzes 207 drückt und hierbei die Rückschlagwirkung der Rückschlagventilanordnung 100 bewirkt oder hierzu beiträgt. Des Weiteren weist die Rückschlagventilanordnung 100 oder deren Ventilkörper 101 eine umlaufende Verschiebescheibe 108 auf, welche im hier dargestellten Ausgangszustand zu einer Anschlagfläche 208 für die Verschiebescheibe 108 beabstandet ist. Die Verschiebescheibe 108 ist mit dem Stift 105 verbunden und dient dem einfacheren, verkantungssicheren Aufbringen der Kraft F auf den Stift 105.

Die Rückschlagventilanordnung 100 der Fig. 1a und 1b befindet sich in Fig. 1a bereits im geschlossenen Zustand, in welchem sie wie ein Rückschlagventil Sterilisationsmittel nicht unbeschränkt durch die in ihr enthaltenen oder von ihr (mit-)gebildeten Strömungswege strömen lässt. Sie befindet sich allerdings noch nicht im "verrasteten Zustand". Ein über den Zulauf 205 in Richtung Ventilkörper 101 strömendes Fluid, etwa ein Sterilisationsgas, kann nur oder im wesentlichen nur über einen Bypass 107 in alle für eine Sterilisierung relevanten Bereiche der an sich bereits geschlossenen Rückschlagventilanordnung 100 eintreten und sich durch den Ventilkörper 101 hindurch verteilen.

Das Fluid kann sich weiterhin entlang einer, vorzugsweise senkrecht zur Stiftaufnahme 103 vorgesehenen Eintrittsöffnung 109 verteilen. Alternativ oder ergänzend kann das Fluid in bestimmten, beispielhaften erfindungsgemäßen Ausführungsformen in das langgestreckte Innere des optional hohl ausgestalteten Stifts 105 eintreten und dieses durch nicht dargestellte Öffnungen in Mantel- und/oder Stirnfläche des Stifts 105 wieder verlassen. Alternativ oder ergänzend ergibt sich der Bypass 107 durch den Abstand zwischen der Abstandfläche 208 und der Verschiebescheibe 108.

**Fig. 1b** zeigt die erfindungsgemäße Rückschlagventilanordnung 100 der Fig. 1a im Betriebszustand, in welchem die Rückschlagventilanordnung 100 als Rückschlagventil wirkt. Der Betriebszustand, hierin auch als Betriebsstellung bezeichnet, ist der aktivierte Zustand, hierin auch als Behandlungszustand bezeichnet.

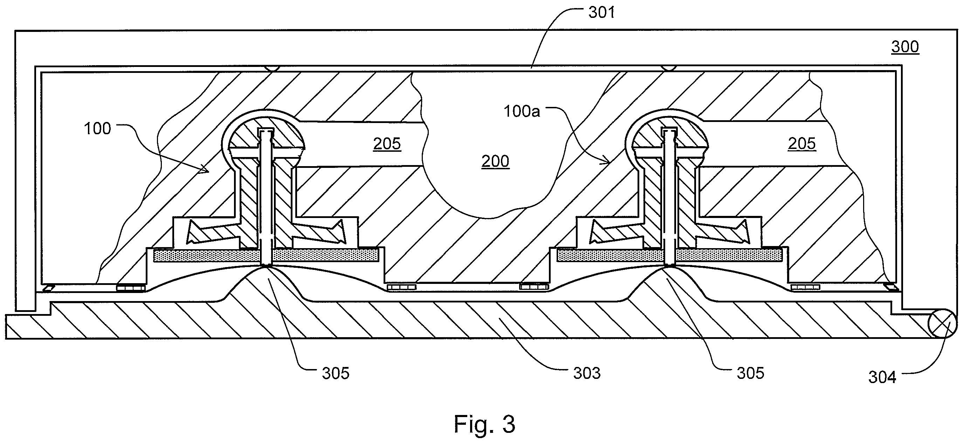

Mittels einer in Pfeilrichtung wirkenden Kraft F, welche vorzugsweise mittels eines Abschnitts, etwa einer Tür 303, der nur in Fig. 3 dargestellten Behandlungsvorrichtung 300 aufgebracht wird, wurde oder wird Druck auf die Verschiebescheibe 108 und den Stift 105 derart ausgeübt, dass dieser tiefer in den Ventilkörper 101 hineingedrückt wird. Dies kann zu einer zumindest vorübergehenden Einbeulung der Folie 203 führen.

Durch das Aufbringen der Kraft F wurde der Stift 105 soweit entlang der Stiftaufnahme 103 in Richtung auf das Innere des Kassettenkörpers 200 verschoben, dass der Bypass 107 nun geschlossen ist. Ein (Arbeits-)Fluid, beispielsweise Blut, ein Medikament oder Substituat, kann die Rückschlagventilanordnung 100 in dem hier dargestellten Betriebszustand, in welchem das Rückschlagventil aktiviert ist, nur unter entsprechend hohem Druck und auch nur in Richtung vom Zulauf 205 auf den Ablauf, nicht aber umgekehrt, durchströmen.

Der Abstand zwischen Abstandfläche 208 und Verschiebescheibe 108 besteht nicht mehr. Beide Elemente berühren einander, was Zeichen dafür ist, dass der Stift 105 ausreichend tief in die Stiftaufnahme 103 hinein verschoben wurde.

Der Stift 103 befindet sich wie im Ausgangszustand der Fig. 1a im Wesentlichen in jenem Abschnitt der Stiftaufnahme 103, welche bezogen auf die Darstellung der Fig. 1b unterhalb der Eintrittsöffnung 109 liegt. Sein Endabschnitt 105a steckt im Betriebszustand in einem Endabschnitt 103a der Stiftaufnahme, welche oberhalb der Eintrittsöffnung 109 liegt bzw. zu erkennen ist.

Der Stift 103 hält in der in Fig. 1b gezeigten Rückschlagposition auch dann noch, wenn die mittels Pfeil angedeutete Kraft F nachlässt oder aufgehoben wird. Man kann hierbei von einer Verrastung des Stifts 105 im Endabschnitt 103a der Stiftaufnahme reden. Eine Verrastung im Sinne der vorliegenden Erfindung liegt dann vor, wenn das die Rückschlagventilanordnung 100 bei Wegfall der Aktivierungskraft F ohne Einwirkung von außerhalb der medizinischen Funktionsvorrichtung 200 im aktivierten Ausgangszustand verbleibt. Dabei ist es unerheblich, ob die Verrastung durch Formschluss und/oder durch Reibschluss erzielt wurde. Bewirkt wird dies im gezeigten Beispiel allein durch einen Reibschluss zwischen Stiftaufnahme 103 und Stift 105. Dabei kann der Reibschluss in Bereichen unterhalb der Eintrittsöffnung 109 vorliegen, er kann alternativ oder ergänzend auch im Endbereich 103a vorliegen. In bestimmten erfindungsgemäßen Ausführungsformen besteht Reibschluss sowohl unter- als auch oberhalb der Eintrittsöffnung 109. Dabei kann der unterhalb der Eintrittsöffnung 109 herrschende Reibschluss dergestalt bestimmt sein, dass der Stift 105 bereits während der Herstellung und bei noch nicht aufgesetzter Folie 200 nicht mehr aus der Stiftaufnahme 103 fallen kann. Dieser Reibschluss muss nicht höher gewählt sein, und je niedriger er ist, desto einfacher ist es, den Stift 105 gegen den bestehenden Reibschluss vom Ausgangszustand in den Betriebszustand zu verschieben. Der Reibschluss, der im Betriebszustand im Endbereich 103a und 105a von Stiftaufnahme und Stift herrscht, kann hieran gemessen höher sein. Er ist jedenfalls so hoch, dass der Reibschluss insgesamt ausreicht, eine Sicherung des Stifts 105 in der Betriebsstellung auch über das Behandlungsende hinaus und auch bei Wegfall der Kraft F sicherzustellen, um die Rückschlagventilanordnung 100 im aktivierten, verrasteten Zustand zu halten.

Alternativ oder ergänzend zum hier beschriebenen Reibschluss, mittels dem der Stift 105 nach seinem Verschieben mittels der Kraft F in der in Fig. 1b gezeigten Stellung in der Stiftaufnahme 103 steckt und welcher den Betriebszustand aufrechterhält, kann letztere durch einen Formschluss erzeugt und aufrecht erhalten werden. So kann eine Schnappverbindung, ein Widerhakensystem oder dergleichen dafür sorgen, dass der Stift 105 in seiner (Betriebs-)Stellung bleibt.

Wie den Fig. 1a und 1b zu entnehmen ist, verbindet die Eintrittsöffnung 109 das Innere des Ventilkörpers 101 über mehrere Öffnungen, wenigstens aber eine Öffnung, mit dessen Äußeren. In Fig. 1a sind exemplarisch zwei solcher Öffnungen gezeigt, welche beispielhaft am linken und rechten Rand des Ventilkörpers 101 angeordnet sind und mittels einer in Fig. 1a waagrechten Verbindung in Fluidkontakt stehen. Das Vorsehen mehrerer Eintrittsöffnungen 109 erlaubt es vorteilhaft auf eine Positionsmarke oder eine strukturelle Kodierung zum Gewährleisten eines korrekten Einbaus des zumeist rotationssymmetrisch ausgestalteten Ventilkörpers zu verzichten.

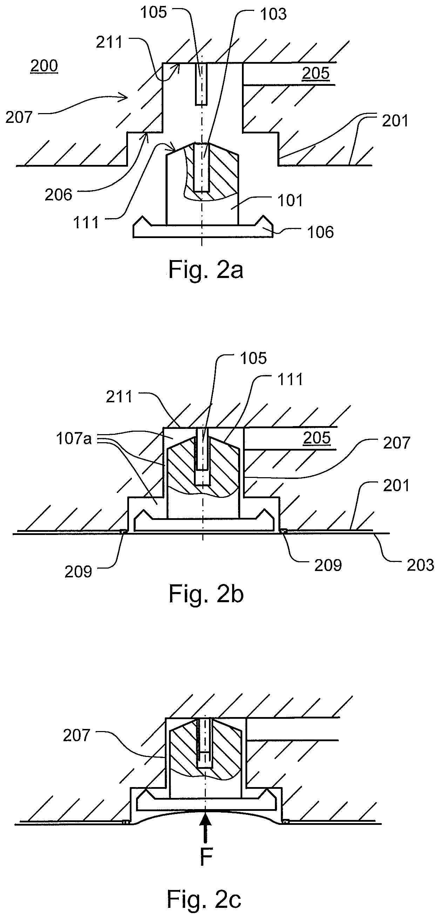

Die in Fig. 1a und 1b gezeigte Rückschlagventilanordnung 100 ist in der in Fig. 1a gezeigten Stellung bereits geschlossen (d. h. die für die Rückschlagwirkung relevanten Komponenten sind bereits in Stellung, um eine Rückschlagwirkung zu erzielen). Die Durchströmung mit Sterilisationsmedium wird durch den Bypass 107 sichergestellt. Von der vorliegenden Erfindung umfasst sind allerdings auch Rückschlagventilanordnungen, welche in jedem unverrasteten (Ausgangs-)Zustand noch ausreichend durchgängig für das Sterilisationsmittel sind. Solche erfindungsgemäßen Ausführungsformen benötigen keinen Bypass. Ein Beispiel einer solchen Ausführungsform zeigen die Fig. 2a bis 2c.

Fig. 2a zeigt eine erfindungsgemäße Rückschlagventilanordnung 100 in einer zweiten Ausführungsform in Explosionsdarstellung.

Die Ausführungsform der Fig. 2a unterscheidet sich von jener der Fig. 1a und 1b u. a. dadurch, dass der Stift 105 Teil des Ventilsitzes 207, nicht aber des Ventilkörpers 101 ist. Die Stiftaufnahme 103 ist hingegen wie auch in Fig. 1a und 1b Teil des Ventilkörpers 101. Sie liegt, wie in der ersten Ausführungsform der Fig. 1a, in einem Inneren des Ventilkörpers und mündet in einer Stirnfläche hiervon. In der hier gezeigten zweiten Ausführungsform dient die Stiftaufnahme 103 als Führungs- und Halteaufnahme, der Stift 105 dient als Führungs- und Haltestift. Die Stiftaufnahme 103 ist exemplarisch als Sackloch ausgestaltet.

Die Ausführungsform der Fig. 2a unterscheidet sich von jener der Fig. 1a und 1b ferner dadurch, dass sie keine Verschiebescheibe 108 und keinen Bypass 107 aufweist.

Der - bezogen auf die Fig. 2a - obere Stirnbereich 111 des Ventilkörpers 101 ist im Zusammenwirken mit dem Aufnahmebereich 211 des Ventilsitzes 207 für den Ventilkörper 101 derart ausgestaltet, dass diese Bauteile bei ihrem Kontakt miteinander eine Dichtung ergeben, wie dies in Fig. 2c gezeigt ist.

Der Ventilkörper 101 ist hier rein beispielhaft aus Kunststoff, insbesondere aus Polycarbonat (kurz: PC), ausgestaltet, die Dichtlippe 106 rein beispielhaft aus Elastomer. Der Ventileinsatz, bestehend aus Ventilkörper 101 und Dichtlippe 106 oder diese aufweisend, ist somit als 2-Komponententeil gefertigt, vorzugsweise gespritzt (2K-Spritzgussteil).

**Fig. 2b** zeigt die Rückschlagventilanordnung 100 der Fig. 2a im Ausgangszustand, dem unverrasteten und - folglich - zudem nicht-aktivierten Sterilisationszustand. In diesem Zustand kann die Rückschlagventilanordnung 100 von Gas oder Flüssigkeit zum Zwecke der Sterilisierung - von einer Rückschlagfunktion unbehindert, da diese noch nicht aktiviert ist - durchströmt werden. Hierzu dient auch der freie (Strömungs-)Weg 107a, welcher sich daraus ergibt, dass der-stets bezogen auf die Fig. 2a - obere Stirnbereich 111 des Ventilkörpers 101 noch keinen Kontakt mit dem Aufnahmebereich 211 des Ventilsitzes 207 für den Ventilkörper 101 hat und diese Bauteile mangels Kontakt miteinander keine Dichtung ergeben. Hierdurch bedingt kann Fluid auch durch einen Spalt strömen, welcher sich zwischen Mantelfläche des Ventilkörper 101 und der inneren Zylinderwand des Ventilsitzes 207 ergibt. Von hier aus kann das Fluid ferner an der noch nicht geschlossenen Dichtlippe 106, welche in Fig. 2b - anders als in Fig. 2c - die Anschlagfläche 206 des Ventilsitzes 207 noch nicht berührt und hierdurch abdichtet, vorbei streichen. Selbstverständlich ist eine Strömung auch in der zur hier genannten Strömungsrichtung entgegen gesetzten Richtung möglich.

Die Folie 203 ist bereits auf den Kassettenkörper 201 aufgebracht und mittels Befestigungen 209 an diesem befestigt, etwa verklebt, verschweißt, usw.

**Fig. 2c** zeigt die Rückschlagventilanordnung der Fig. 2a und 2b im Betriebszustand, in welchem der Ventilkörper 101 aufgrund der wiederum per Pfeil angedeuteten Kraft F mittels der Stift-Stiftaufnahme-Verbindung derart mit dem Ventilsitz 207 verbunden ist oder wurde (also sowohl "aktiviert" als auch "verrastet" wurde), dass der Ventilkörper 101 auch nach Wegfall der o. g. Kraft nicht seine Rückschlagfunktion aufgeben würde.

Der freie Weg 107a ist in der in Fig. 2b gezeigten Stellung des Ventilkörpers 101 zum Ventilsitz 207 geschlossen. Fluide können nur noch bei entsprechend hohem Druck und in der vorgegebenen Richtung die Rückschlagventilanordnung 100 durchströmen. Die Rückschlagventilanordnung 100 dient damit im aktivierten Zustand, dem Betriebszustand einer Abdichtung der medizinischen Funktionsvorrichtung 200 selbst nachdem diese von der Blutbehandlungsvorrichtung gelöst oder aus dieser entnommen wurde.

**Fig. 3** zeigt schematisch stark vereinfacht eine Draufsicht auf eine Blutbehandlungsvorrichtung 300. Sie weist einen Aufnahmeabschnitt 301 zur Aufnahme einer medizinischen Funktionsvorrichtung 200, hier eine Blutkassette, auf. Ferner weist sie eine bewegbare Begrenzungsvorrichtung 303, hier eine Tür, auf. Die im Schnitt dargestellte Tür ist mittels eines Scharniers 304 an der Blutbehandlungsvorrichtung 300 befestigt und kann mittels dieses von der nicht in Fig. 3 gezeigten geöffneten Stellung, in welcher die Blutkassette 200 aus der Blutbehandlungsvorrichtung 300 entnommen werden kann, in die hier gezeigte Stellung verschwenkt werden, in welcher der Aufnahmeabschnitt 301 geschlossen ist.

Die Blutkassette 200 weist neben der aus den vorangegangenen Figuren bereits bekannten Rückschlagventilanordnung 100 eine zweite Rückschlagventilanordnung 100a auf.

Die Begrenzungsvorrichtung 303 bzw. Tür weist zwei Aktivierungsstössel 305 auf. Diese sind angeordnet, um beim Schließen der Tür die oben mit F bezeichnete Aktivierungskraft gleichzeitig auf beide Ventilkörper 101 oder beide Stifte 105 der Rückschlagventilanordnungen 100 und 100a aufzubringen.

Die Anzahl der in Fig. 3 gezeigten Rückschlagventilanordnungen 100, 100a ist rein exemplarisch. Es können mehr oder weniger Rückschlagventilanordnungen vorgesehen sein.

Die Befestigung der Begrenzungsvorrichtung 303 bzw. Tür an der Blutbehandlungsvorrichtung 300 ist ebenfalls beispielhaft als Scharnierverbindung gezeigt. Jeder andere Mechanismus, mittels welchem ein oder mehrere Rückschlagventilanordnungen, vorzugsweise automatisch, vorzugsweise gleichzeitig, aktiviert werden können, sind von der vorliegenden Erfindung mit umfasst.

### Bezugszeichen

- 100: Rückschlagventilanordnung
- 100a: zweite Rückschlagventilanordnung
- 101: Ventilkörper
- 103: Stiftaufnahme
- 103a: Endabschnitt
- 105: Stift
- 105a: Endabschnitt
- 106: Dichtlippe
- 107: Bypass
- 107a: freier Weg
- 108: Verschiebescheibe
- 109: Eintrittsöffnung
- 111: Stirnbereich des Ventilkörpers

- 200: medizinische Funktionsvorrichtung, Blutkassette
- 201: Kassettenkörper
- 203: Folie
- 205: Zulauf
- 206: Anschlagfläche
- 207: Ventilsitz
- 208: Anschlagfläche
- 209: Befestigungen
- 211: Aufnahmebereich

- 300: Blutbehandlungsvorrichtung
- 301: Aufnahmeabschnitt
- 303: bewegbare Begrenzungsvorrichtung, Tür
- 304: Scharnier
- 305: Aktivierungsstössel

## Patentansprüche

1. Rückschlagventilanordnung (100) für eine Blutkassette oder einen Blutschlauchsatz, mit einem Zulauf (205) und einem Ablauf für medizinische Fluide, mit einem Ventilkörper (101), wobei die Rückschlagventilanordnung (100) in einem Ausgangszustand ein Durchströmen eines Arbeitsfluids und/oder eines Sterilisationsfluids durch die Rückschlagventilanordnung (100) in beiden Durchströmungsrichtungen, also vom Zulauf (205) zum Ablauf und umgekehrt, zulässt, und wobei die Rückschlagventilanordnung (100) in einem Betriebszustand nur eine Durchströmungsrichtung des Arbeitsfluids zulässt, **dadurch gekennzeichnet, dass** die Rückschlagventilanordnung (100) eine Stift-Stiftaufnahme-Verbindung aufweist, welche einen Stiftaufnahmeabschnitt zum Aufnehmen eines Stiftes und den im Stiftaufnahmeabschnitt bewegbar angeordneten Stift aufweist, wobei die Rückschlagventilanordnung (100) durch Kraftaufbringung auf die Stift-Stiftaufnahme-Verbindung und/oder durch eine Wegeinprägung auf die Stift-Stiftaufnahme-Verbindung, jeweils in Längsrichtung von Stift und Stiftaufnahme, vom Ausgangszustand in den Betriebszustand überführbar ist.

2. Rückschlagventilanordnung (100) nach Anspruch 1, wobei die Rückschlagventilanordnung (100) im Ausgangszustand einen Bypass (107) zum Durchströmen des Sterilisationsfluids aufweist.

3. Rückschlagventilanordnung (100) nach Anspruch 2, wobei die Stiftaufnahme (103) der Stift-Stiftaufnahme-Verbindung der Bypass (107) ist oder zumindest bereichsweise den Bypass (107) umfasst oder bildet.

4. Rückschlagventilanordnung (100) nach Anspruch 2 oder 3, wobei der Stift (105) der Stift-Stiftaufnahme-Verbindung innen hohl und zumindest bereichsweise den Bypass (107) umfasst oder bildet.

5. Rückschlagventilanordnung (100) nach einem der Ansprüche 2 bis 4, wobei der Stift (105) der Stift-Stiftaufnahme-Verbindung zumindest bereichsweise auf seinem äußeren Umfang gegen den Ventilkörper (101) abdichtet.

6. Rückschlagventilanordnung (100) nach einem der vorangegangenen Ansprüche, wobei der Ventilkörper (101) sowohl den Stift (105) als auch die Stiftaufnahme (103) aufweist.

7. Rückschlagventilanordnung (100) nach einem der vorangegangenen Ansprüche, wobei die Rückschlagventilanordnung (100) im Betriebszustand eine reibschlüssige Verbindung des Stifts (105) mit wenigstens einem Abschnitt der Stiftaufnahme (103) aufweist.

8. Rückschlagventilanordnung (100) nach einem der vorangegangenen Ansprüche, wobei der Ventilkörper (101) einen ersten Bereich mit Stiftaufnahme (103) für den Stift und einen zweiten Bereich zum Abdichten eines Strömungswegs des Arbeitsfluids im Betriebszustand aufweist, wobei der zweite Bereich flexibler oder weicher als der erste Bereich ausgestaltet ist.

9. Rückschlagventilanordnung (100) nach einem der vorangegangenen Ansprüche, wobei der Ventilkörper (101) oder ein Ventileinsatz aus Kunststoff hergestellt ist oder Kunststoff aufweist.

10. Rückschlagventilanordnung (100) nach einem der vorangegangenen Ansprüche, wobei der Ventilkörper (101) oder der Ventileinsatz als 2-Komponenten-Spritzgußteil hergestellt ist.

11. Rückschlagventilanordnung (100) nach einem der vorangegangenen Ansprüche, wobei die Stift-Stiftaufnahme-Verbindung ausgestaltet ist, um die Rückschlagventilanordnung (100) mittels Reibschluss und/oder Formschluss im Betriebszustand zu halten.

12. Medizinische Funktionsvorrichtung mit wenigstens einer Rückschlagventilanordnung (100) nach einem der vorangegangenen Ansprüche.

13. Medizinische Funktionsvorrichtung nach Anspruch 12, wobei die Rückschlagventilanordnung (100) einen Ventilsitz (207) und optional eine Folie (203) zum Abdichten des Ventilsitzes (207), des Arbeitsfluids und/oder des Sterilisationsfluids gegen die Umgebung aufweist.

14. Medizinische Funktionsvorrichtung nach einem der Ansprüche 12 bis 13, wobei der Stift (105) mit dem Ventilsitz (207) verbunden oder Teil hiervon ist, und wobei die Stiftaufnahme (103) relativ zu dem Stift (105) verschiebbar und Teil des Ventilkörpers (101) ist.

15. Medizinische Funktionsvorrichtung nach einem der Ansprüche 12 bis 14 mit wenigstens zwei Rückschlagventilanordnungen (100) gemäß einem der Ansprüche 1 bis 11, welche angeordnet sind, um gleichzeitig aus dem Ausgangszustand in den Betriebszustand überführbar zu sein.

16. Blutbehandlungsvorrichtung (300), vorzugsweise Dialysiervorrichtung, mit einem Aufnahmeabschnitt (301) zur Aufnahme einer medizinischen Funktionsvorrichtung (200) nach einem der Ansprüche 12 bis 14, wobei die Blutbehandlungsvorrichtung (300) eine bewegbare Begrenzungsvorrichtung (303) zum Begrenzen des Aufnahmeabschnitts (301) aufweist, wobei die Begrenzungsvorrichtung (303) ausgestaltet ist, um durch ihre Bewegung eine oder alle Rückschlagventilanordnungen (100) der in den Aufnahmeabschnitt (301) aufgenommenen medizinischen Funktionsvorrichtung (200) aus dem Ausgangszustand in den Betriebszustand zu überführen.

## Claims

1. A check valve arrangement (100) for a blood cassette or a blood tubing set, comprising an inlet (205) and an outlet for medical fluids, with a valve body (101),
wherein, in an initial state, the check valve arrangement (100) permits a flow of a working fluid and/or of a sterilisation fluid through the check valve arrangement (100) in both flow directions, i.e. from the inlet (205) to the outlet and vice versa,
and wherein, in an operating state, the check valve arrangement (100) permits only one direction of flow of the working fluid,
**characterized in that** the check valve arrangement (100) comprises a pin-pin-reception connection comprising a pin-reception portion for receiving a pin and the pin movably arranged in the pin-reception portion,
wherein the check valve arrangement (100) can be transferred from the initial state into the operating state by force application on the pin-pin-reception connection, and/or by an impression of path on the pin-pin-reception connection, each in longitudinal direction of the pin and the pin-reception.

2. The check valve arrangement (100) according to claim 1, wherein the check valve arrangement (100), in the initial state, comprises a bypass (107) for flowing the sterilisation fluid through.

3. The check valve arrangement (100) according to claim 2, wherein the pin-reception (103) of the pin-pin-reception connection is the bypass (107) or includes or forms the bypass (107) at least in some sections.

4. The check valve arrangement (100) according to claim 2 or 3, wherein the pin (105) of the pin-pin-reception connection is hollow in the inside and includes or forms the bypass (107) at least in some sections.

5. The check valve arrangement (100) according to anyone of claims 2 to 4, wherein the pin (105) of the pin-pin-reception connection seals on its outer periphery against the valve body (101) at least in some sections.

6. The check valve arrangement (100) according to anyone of the preceding claims, wherein the valve body (101) comprises both the pin (105) as well as the pin-reception (103).

7. The check valve arrangement (100) according to anyone of the preceding claims, wherein the check valve arrangement (100) in the operating state comprises a frictional closure connection of the pin (105) with at least a portion of the pin-reception (103).

8. The check valve arrangement (100) according to anyone of the preceding claims, wherein the valve body (101) comprises a first region with a pin-reception (103) for the pin and a second region for sealing a flow path of the working fluid in the operating state, the second region being designed to be more flexible or softer than the first region.

9. The check valve arrangement (100) according to anyone of the preceding claims, wherein the valve body (101) or a valve insert is made of plastic or comprises plastic.

10. The check valve arrangement (100) according to anyone of the preceding claims, wherein the valve body (101) or the valve insert is produced as a 2-components injection moulded part.

11. The check valve arrangement (100) according to anyone of the preceding claims, wherein the pin-pin-reception connection is designed to hold the check valve arrangement (100) in the operating state by means of frictional and/or form closure.

12. A medical functional apparatus comprising at least one check valve arrangement (100) according to anyone of the preceding claims.

13. The medical functional apparatus according to claim 12, wherein the check valve arrangement (100) comprises a valve seat (207) and optionally a film (203) for sealing the valve seat (207), the working fluid and/or the sterilization fluid against the environment.

14. The medical functional apparatus according to anyone of claims 12 to 13, wherein the pin (105) is connected with or part of the valve seat (207), and wherein the pin-reception (103) is slidable relative to the pin (105) and part of the valve body (101).

15. The medical functional apparatus according to anyone of claims 12 to 14 comprising at least two check valve arrangements (100) according to anyone of claims 1 to 11, which are arranged to be simultaneously transferable from the initial state to the operating state.

16. A blood treatment apparatus (300), preferably a dialysis apparatus, comprising a reception portion (301) for receiving a medical functional apparatus (200) according to anyone of claims 12 to 14, wherein the blood treatment apparatus (300) comprises a movable limiting apparatus (303) for limiting the reception portion (301), the limiting apparatus (303) being designed to transfer, by its movement, one or all check valve arrangement(s) (100) received in the reception portion (301) of the medical functional apparatus (200) from the initial state to the operating state.

## Revendications

1. Un agencement de soupape anti-retour (100) pour une cassette à sang ou un set de tuyaux sanguins, comprenant une entrée (205) et une sortie pour des fluides médicaux, avec un corps de soupape (101),
où, dans un état initial, l'agencement de soupape anti-retour (100) permet l'écoulement d'un fluide de travail et/ou d'un fluide de stérilisation au travers de l'agencement de soupape anti-retour (100), dans les deux sens d'écoulement, c'est-à-dire de l'entrée (205) vers la sortie et inversement, et où, dans un état de fonctionnement, l'agencement de soupape anti-retour (100) ne permet qu'un seul sens d'écoulement du fluide de travail,
**caractérisé en ce que** l'agencement de soupape anti-retour (100) comprend une liaison broche-réceptacle de broche comprenant une section réceptacle de broche destinée à recevoir une broche ainsi que la broche agencée de manière mobile dans la section du réceptacle de broche,
où l'agencement de soupape anti-retour (100) peut être transféré de l'état initial à l'état de fonctionnement par application d'une force sur la liaison broche-réceptacle de broche et/ou par impression d'une trajectoire sur la liaison broche-réceptacle de broche, respectivement dans la direction longitudinale de la broche et du réceptacle à broche.

2. L'agencement de soupape anti-retour (100) selon la première revendication, où l'agencement de soupape anti-retour (100), à l'état initial, comprend un by-pass (107) pour permettre l'écoulement du fluide de stérilisation au travers de celui-ci.

3. L'agencement de soupape anti-retour (100) selon la revendication 2, où le réceptacle de broche (103) de la liaison broche-réceptacle de broche est le by-pass (107) ou inclut ou forme le by-pass (107) au moins dans certaines sections.

4. L'agencement de soupape anti-retour (100) selon la revendication 2 ou 3, où la broche (105) de la liaison broche-réceptacle de broche est creuse à l'intérieur et inclut ou forme le by-pass (107) au moins dans certaines sections.

5. L'agencement de soupape anti-retour (100) selon l'une quelconque des revendications 2 à 4, où la broche (105) de la liaison broche-réceptacle de broche assure sur sa périphérie externe l'étanchéité contre le corps de soupape (101) au moins dans certaines sections.

6. L'agencement de soupape anti-retour (100) selon l'une quelconque des revendications précédentes, où le corps de soupape (101) comprend à la fois la broche (105) et le réceptacle de broche (103).

7. L'agencement de soupape anti-retour (100) selon l'une quelconque des revendications précédentes, où l'agencement de soupape anti-retour (100) en état de fonctionnement comprend une liaison par friction de la broche (105) avec au moins une section du réceptacle de broche (103).

8. L'agencement de soupape anti-retour (100) selon l'une quelconque des revendications précédentes, où le corps de soupape (101) comprend une première zone avec un réceptacle de broche (103) pour la broche ainsi qu'une seconde zone pour assurer l'étanchéité d'une trajectoire d'écoulement du fluide de travail en état de fonctionnement,
la seconde zone étant conçue pour être plus flexible ou plus souple que la première zone.

9. L'agencement de soupape anti-retour (100) selon l'une quelconque des revendications précédentes, où le corps de soupape (101) ou un insert de soupape est en matière plastique ou comprend du plastique.

10. L'agencement de soupape anti-retour (100) selon l'une quelconque des revendications précédentes, où le corps de soupape (101) ou l'insert de soupape est réalisé sous forme de pièce moulée par injection à 2 composants.

11. L'agencement de soupape anti-retour (100) selon l'une quelconque des revendications précédentes, où la liaison broche-réceptacle de broche est conçue pour maintenir l'agencement de soupape anti-retour (100) en état de fonctionnement au moyen de friction et/ou de complémentarité de forme.

12. Un appareil de fonctionnement médical comprenant au moins un agencement de soupape anti-retour (100) selon l'une quelconque des revendications précédentes.

13. L'appareil de fonctionnement médical selon la revendication 12, où l'agencement de soupape anti-retour (100) comprend un siège de soupape (207) et de façon facultative un film (203) pour assurer l'étanchéité du siège de soupape (207), du fluide de travail et/ou du fluide de stérilisation contre l'environnement.

14. L'appareil de fonctionnement médical selon l'une quelconque des revendications 12 à 13, où la broche (105) est reliée au siège de soupape (207) ou en fait partie, et où le réceptacle de broche (103) peut coulisser par rapport à la broche (105) et fait partie du corps de soupape (101).

15. L'appareil de fonctionnement médical selon l'une des revendications 12 à 14 comprenant au moins deux agencements de soupape anti-retour (100) selon l'une quelconque des revendications 1 à 11, qui sont agencés de manière à pouvoir être transférés simultanément de l'état initial à l'état de fonctionnement.

16. Un appareil de traitement du sang (300), de préférence un appareil de dialyse, comprenant une section de réceptacle (301) destinée à recevoir un appareil de fonctionnement médical (200) selon l'une quelconque des revendications 12 à 14, où l'appareil de traitement du sang (300) comprend un appareil de restriction mobile (303) destiné à restreindre la section de réceptacle (301), l'appareil de restriction (303) étant conçu pour transférer, de par son mouvement, un ou tous les agencement(s) de soupape anti-retour (100) de l'appareil de fonctionnement médical (200) réceptionné(s) dans la section de réceptacle (301), de l'état initial à l'état de fonctionnement.
